# EUROPEAN PATENT APPLICATION

(11) **EP 1 106 186 A2**
(43) Date of publication of application: **13.06.2001**
(21) Application number: 00310690.3
(22) Date of filing: 01.12.2000
(51) Int. Cl.: A61K 49/00

(54) **Non-covalent bioconjugates useful for magnetic resonance imaging**

(30) Priority: 08.12.1999 US 457347
(71) Applicant: Mallinckrodt Inc., St. Louis, Missouri 63134 (US)
(72) Inventor: Rajagopalan, Raghavan, Maryland Heights, MO 63043 (US); Bugaj, Joseph Edward, St.Charles, MO 63303 (US); Dorshow, Richard Bradley, St. Louis, MO 63146 (US); Achilefu, Samuel, Bridgeton, MO 63044 (US)
(74) Representative: Jones, Alan John

(57) **Abstract**

A composition having non-covalent carrier-hapten bioconjugates of the formula:

HM ----- CM

wherein HM is a hapten molecule whose molecular weight is generally, but not always, less than 1000 Daltons and is capable of performing specific functions; CM is a carrier molecule, whose molecular weight is generally, but not always, more than 1000 Daltons and is capable of transporting the hapten to a specific site; and the dashed line is a non-covalent bond between the carrier molecule and the hapten molecule. Preferably, the bioconjugates are formed from a paramagnetic metal complex and a carrier molecule such as methylated serum albumin, polyarginine, lysine-phenylalanine copolymer, polyacrylamide-co-diallyldimethylammonium, arginine-serine copolymer, and styrene-maleic acid copolymer.

## Description

### BACKGROUND OF THE INVENTION

### Field of the Invention

This invention pertains to novel non-covalent carrier-hapten bioconjugates and their use in delivering diagnostic and therapeutic agents selectively to target tissues and organs. Particularly, this invention pertains to the selective delivery of paramagnetic non-covalent carrier-hapten bioconjugates to a specific site for the purpose of determining the structure and function of tissues or organs using magnetic resonance (MR) imaging procedures.

### Description of the Prior Art

Non-covalent intermolecular forces (e.g., electrostatic, hydrogen bonding and Van der Waals interactions) play a vital role in many biological processes such as enzyme catalysis, drug-receptor interaction, antigen-antibody interaction, biotin-avidin interaction, DNA double helix formation, phagocytosis, pigmentation in plants and animals, and cellular transport. Many of the wide variety of colors observed in flowers and plants, for instance, are attributed to non-covalent association between the natural pigments and carbohydrates or proteins found in plant cells.

Non-covalent forces can alter the physicochemical and/or biological properties of haptens or carriers. For example, human serum albumin binds various molecules in a non-selective fashion and facilitates the transport of these molecules across the vasculature to the cells. Association of dyes or pigment molecules with proteins or carbohydrates usually changes the chemical or photo stability, changes the intensity and/or the wavelength of absorption/emission maxima, or both. Association of various paramagnetic gadolinium complexes to serum albumin has been shown to enhance the relaxivity of water protons by a factor of two (T.J. Brady and R.B. Lauffer, *Hepatobiliary NMR Contrast Agents,* Patent Application, **1986**, WO 8606605).

In non-covalent interactions, although the interaction energy per unit interaction is quite small (less than 40 kJ/interaction), the cumulative effect of multiple points of interaction along two surfaces can be substantial and can lead to strong binding between a hapten and a carrier. This approach has been successfully used to prepare anti-DNA antibodies. DNA is a highly charged anionic macromolecule that is normally non-immunogenic; but, when it is complexed with a highly charged cationic methylated bovine serum albumin (MBSA), DNA becomes immunogenic. A non-covalent DNA-MBSA bioconjugate is stable enough to elicit immune response toward DNA. It is clear from the examples above that noncovalently attached bioconjugates function biologically as a single unit. In addition to the above mentioned properties, the carrier molecule may also protect some haptens from chemical, photochemical, or radiolytic degradation.

The present invention is intended to exploit the concept of non-covalent interactions for the design of novel non-covalent bioconjugates for diagnosis using MR imaging procedures. Magnetic resonance contrast agents are widely used in diagnostic medicine. In conventional proton MRI, increased contrast of internal organs and tissues may be obtained by administering compositions containing a paramagnetic substance that increases the relaxation rate of water protons in its vicinity compared to the bulk water. In general, paramagnetic species such as ions of elements with atomic number 21 to 29, 42-44, and 58-70 have been found to be effective as MRI contrast agents. Examples of suitable ions include Cr(III), Mn(II and III), Fe(II and III), Co(II), Ni(II), Cu(II), Pr(III), Nd(III), Sm(III), Yb(III), Gd(III), Tb(III), Dy(III), Ho(III), and Er(III). Because of its high magnetic moment and short electronic correlation time, Gd(III) is the most preferred metal ion in the field of MRI contrast agents. It is always desirable and often necessary to increase the relaxivity of the metal complexes as much as possible. This increase in relaxivity has several benefits which include improvement in resolution, better contrast, reduced toxicity due to lower administered doses, or any combination of these factors. Furthermore, considerable increase in relaxation rate of surrounding water protons may be necessary to image organs, structures, and tissues that only receive a small percentage of the dose or that may be subject to high levels of background interference including motion artifacts. Recently, it was shown that covalent as well as non-covalent attachment of gadolinium complexes containing covalently attached albumin binding groups to serum albumin resulted in a four-fold increase in relaxivity as a result of a decrease in rotational correlation time brought about by strong binding of these complexes to albumin (B.M. Hofman et al., *Blood Pool Agent Strongly Improves 3D Magnetic Resonance Coronary Angiography Using an Inversion Pre-pulse,* Magnetic Resonance in Medicine, **1999**, 360-367; R. B. Lauffer et al., *Diagnostic Imaging Contrast Agents With Extended Blood Retention,* Patent Application, **1996**, WO 9623526; S.R. Woulfe, US Patent, **1999**, 5,888,476). However, the major disadvantage of these approaches is that they not only require conventional bioconjugate chemistry to attach the haptens to carriers, but, more importantly, they require the availability of strong albumin binding groups, which severely limits the range of compounds that may exhibit desirable albumin binding properties.

In conventional bioconjugate chemistry, conjugates are prepared by covalent attachment of various effector molecules such as drugs, hormones, radiopharmaceutical agents, magnetic resonance imaging agents, chemotherapeutic agents, and the like to bioactive carriers. Such a process often involves cumbersome chemical manipulation of the two components, in addition to the complicated synthesis of appropriate activated haptens necessary for covalent attachment. Moreover, the bioactivity of the resulting conjugate is, in many cases, either greatly diminished or obviated altogether. Although the compounds **1** and **2** described by Lauffer 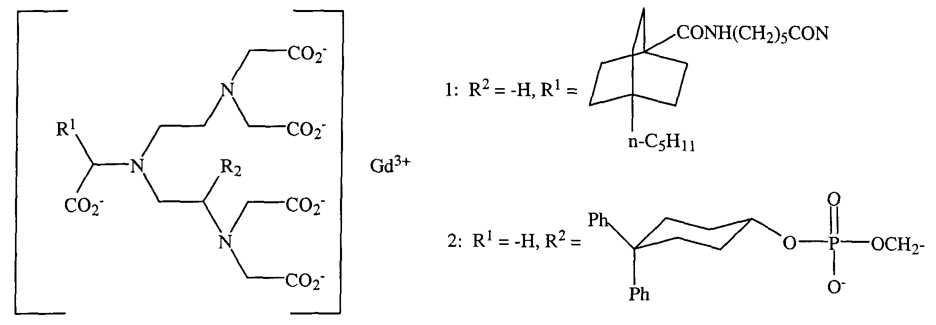 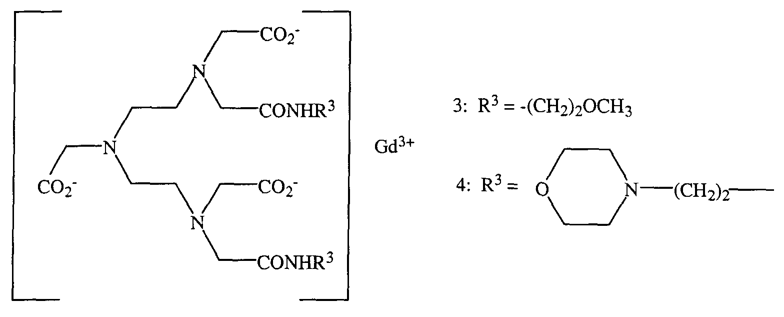 (R. B. Lauffer et al., *Diagnostic Imaging Contrast Agents With Extended Blood Retention,* Patent Application, **1996**, WO 962352); and Woulfe (S.R. Woulfe, Magnetic Resonance Blood Pool Agents, US Patent, **1999**, 5,888,476) bind to albumin in a noncovalent manner, their preparation requires first the covalent attachment of a special albumin binding group to the paramagnetic complex, which resulted in a complex synthetic procedure to prepare and attach this unit to the paramagnetic complex. Moreover, this process relies almost exclusively on the binding ability of that moiety to albumin, whereas in the present invention no such restriction is required. Indeed, the examples described later in the text indicate that the relaxivities of novel bioconjugates formed from **1** or **2** with a lysine-phenylalanine copolymer is higher than the corresponding albumin bioconjugates. Furthermore, compound **3**, which lacks a specific albumin binding group, shows a higher relaxivity when noncovalently associated with a lysine-phenylalanine copolymer as compared to the free complex. Finally, compound **4**, which has a tertiary amine side chain, binds so strongly to polyarginine that it results in formation of a solid precipitate. These observations provide further evidence that strong noncovalent binding can occur between a carrier and an effector molecule leading to a bioconjugate that functions as a single unit. Thus, there is a need for novel paramagnetic bioconjugates that are simple to prepare, exhibit high relaxivity, have easily modifiable binding strengths, and are stable enough to be useful as MRI agents.

WO-A-99 51284 discloses a composition comprising non-covalent carrier-hapten bioconjugates having the formula:

HM ----- CM

wherein HM is a hapten molecule selected from the group consisting of peptides, carbohydrates, photosensitizers, and fluorescent dyes; CM is a carrier molecule selected from the group consisting of proteins, glycoproteins, polypeptides, polysaccharides, inclusion compounds, polynucleotides, lipoproteins, and surfactants; and the dashed line is a non-covalent bond between the carrier molecule and the hapten molecule.

### SUMMARY OF THE INVENTION

It is, therefore, an object of the present invention to provide novel compositions for increasing the relaxivity of paramagnetic complexes via non-covalent association of paramagnetic agents to selected carrier molecules.

It is a further object of the present invention to provide a method for performing a MR diagnostic procedure on a patient.

These objectives are achieved using new and structurally diverse non-covalent carrier-hapten bioconjugates having the formula:

HM ----- CM

wherein HM is a hapten molecule whose molecular weight is generally, but not always, less than 1000 Daltons and is capable of performing specific functions; CM is a carrier molecule, whose molecular weight is generally, but not always, more than 1000 Daltons and is capable of transporting the hapten to a specific site; and the dashed line represents one or more non-covalent bonds between the carrier molecule and the hapten molecule.

Preferably, the bioconjugates are formed from paramagnetic agents and carrier molecules selected from the group consisting of serum albumin, methylated serum albumin, polypeptides with molecular weight ranges from 2000 to 200000 Daltons, polysaccharides with molecular weight ranges from 2000 to 200000 Daltons, polynucleotides with molecular weight ranges from 2000 to 100000 Daltons, cyclodextrins, calixarenes, surfactants, and other natural or synthetic polymeric substances with molecular weights from 2000 to 200000 Daltons.

Most preferably, the bioconjugates are formed from paramagnetic agents selected from the group consisting of paramagnetic metal complexes such as lanthanide, manganese, and iron complexes; super paramagnetic iron oxide particles; or organic free radicals such as nitroxyl radicals whose carbon containing portions contain 1 to 20 carbon atoms; and cationic carrier molecules selected from the group consisting of methylated serum albumin, polyarginine, polylysine, polyornithine, and mixed polyamino acids such as 1:1 poly(lysine/phenylalanine), 3:1 poly (arginine/serine) and the like.

The multiple points of interaction between the hapten and the carrier essentially immobilize the paramagnetic hapten molecule thereby decreasing the rotational correlation time of the paramagnetic agent. Hence, the bioconjugates of this invention are useful as diagnostic agents in medical procedures because they have substantially increased relaxivity compared to free paramagnetic agents and are stable.

Other objects, advantages, and novel features of the present invention will become apparent in the following detailed description of the invention.

### DETAILED DESCRIPTION OF THE INVENTION

The present invention provides new and structurally diverse non-covalent carrier-hapten bioconjugates having the formula:

HM ----- CM

wherein HM is a hapten molecule whose molecular weight is generally, but not always, less than 1000 Daltons and is capable of performing specific functions; CM is a carrier molecule, whose molecular weight is generally, but not always, more than 1000 Daltons and is capable of transporting the hapten to a specific site; and the dashed line represents one or more non-covalent bonds between the carrier molecule and the hapten molecule.

The hapten is either a small molecule or a macromolecule and is selected from the group consisting of peptides, carbohydrates, fluorescent dyes, and paramagnetic agents. The hapten can also be a molecule such as a hormone, antibody, anti-neoplastic agent, enzyme, coenzyme, peptidomimetic, glycomimetic, cell adhesion molecule, radionuclide metal complex, magnetic resonance imaging agent, X-ray opacification agent, or echogenic agent. Perfluorocarbons with a carbon containing portion with 1 to 20 carbon atoms can also be used as haptens.

Preferably, the hapten is a charged or neutral paramagnetic agent such as a paramagnetic metal complex containing either anionic residues such as carboxylate, sulfonate, or phosphonate groups capable of forming non-covalent ion-pair association with cationic carriers; cationic residues such as ammonium or phosphonium groups capable of forming non-covalent ion-pair associations with anionic carriers; lipophilic groups, e.g., lipophilic polyamino acids, capable of binding with lipophilic portions of the carrier molecule via dispersion forces; or neutral electron pair donors (Lewis bases) such as amino groups capable of forming hydrogen bonding association with carriers containing hydrogen donor groups such as carboxylic acids or alcohols. Most preferably, the hapten is a negatively charged paramagnetic gadolinium, manganese, or iron complex containing anionic lipophilic residues.

The carrier is selected from the group consisting of proteins such as albumins and globulins, glycoproteins, polypeptides, polysaccharides, polynucleotides, lipoproteins, surfactants, polycarboxylic acids, polyamines, polyamides, polyalcohols, polyethers and other natural or synthetic polymeric substances.

Preferably, the carrier is a macromolecule selected from the group consisting of proteins such as albumin or methylated albumin, glycoproteins such as antibodies or selectins, polysaccharides such as inulin or lectins, polynucleotides such as DNA or RNA or polymers such as polyadenylate, polyuridylate, polythymidylate, polyguanylate and polycytidylate, inclusion agents such as cyclodextrins or calixarenes, polyamino acids with one or many different amino acid types, synthetic polymers containing charged groups and aromatic rings, and surfactants such as Tween. Most preferably the carrier is a polymeric amino acid containing one or more of the amino acids arginine, lysine, histidine, serine or phenylalanine, or is a synthetic polymer containing styrene and quaternary ammonium groups.

In a preferred embodiment, a bioconjugate consists of a paramagnetic metal complex hapten and a polycationic carrier selected from the group consisting of polyarginine and a 1:1 polymer of lysine and phenylalanine wherein the hapten and the carrier are held together by non-covalent forces.

In another preferred embodiment, a bioconjugate consists of a paramagnetic metal complex hapten and a polyanionic carrier selected from the group consisting of polyacrylate, polyaspartate, polycarboxylate and polyglutamate wherein the hapten and the carrier are held together by non-covalent forces.

In another preferred embodiment, a bioconjugate consists of a paramagnetic metal complex hapten and a neutral carrier selected from the group consisting of polysaccharide, polyserine, and polyalcohol acids wherein the hapten and the carrier are held together by non-covalent forces. Polyacrylamide-co-allyldimethylamine copolymer is one example of a neutral carrier which can be used.

The paramagnetic agents as well as the carriers of the present invention may vary widely depending on the contemplated application. For blood persistent agents high molecular weight (>50,000 Daltons) polymers are preferable. For renal function measurements, a polysaccharide or anionic polypeptide is desirable.

The paramagnetic complexes **1-4** employed in this invention have been described previously in the following references which are incorporated herein by reference (R. B. Lauffer et al., *Diagnostic Imaging Contrast Agents With Extended Blood Retention,* Patent Application, **1996**, WO 9623526; and S.R. Woulfe, **1999**, *Magnetic Resonance Blood Pool Agents,* US Patent 5,888,476; C.F.G.C. Geraldes et al., *Preparation, Physico-Chemical Characterization, and Relaxometry Studies of Various Gadolinium-DTPA-Bis(Amide) Derivatives as Potential Magnetic Resonance Contrast Agents,* Magnetic Resonance Imaging, **1995**, 13(3), 401-420). The non-covalent carrier-hapten bioconjugates of this present invention can be advantageously prepared by simply mixing the two components in an optimal stoichiometric proportion and administering an effective amount of this mixture contained in a pharmaceutically acceptable formulation into an individual either systemically or locally to the organ or tissue to be studied. Alternatively, the bioconjugates can be isolated and stored by methods well known in the art. The novel bioconjugates of the present invention have broad clinical utility, which includes, but is not limited to, diagnostic imaging of tumors, inflammation (both sterile and bacterial), impaired vasculature, and myocardial viability.

The novel bioconjugates of this invention can be formulated into diagnostic compositions for enteral, parenteral, or oral administration. These compositions contain an effective amount of bioconjugate along with conventional pharmaceutical carriers and excipients appropriate for the type of administration contemplated. These compositions may also include stabilizing agents such as ascorbic or gentisic acid. For example, parenteral compositions advantageously contain a sterile aqueous solution or suspension of bioconjugates whose concentration ranges from about 1 µM to about 1 M. Preferred parenteral formulations have a concentration of bioconjugates of 100 µM to 1 M. Such solutions also may contain pharmaceutically acceptable buffers and, optionally, electrolytes such as sodium chloride. Formulations for enteral administration may vary widely as is well-known in the art. In general, such formulations are liquids which include an effective amount of bioconjugates in aqueous solution or suspension. Such enteral composition may optionally include buffers, surfactants, thixotropic agents, and the like. Compositions for oral administration may also contain flavoring agents and other ingredients for enhancing their organoleptic qualities.

The diagnostic compositions are administered in doses effective to achieve the desired diagnostic objective. Such doses may vary widely depending upon the particular bioconjugates employed, the organs or tissues to be examined, the equipment employed in the clinical procedure, and the like.

The present invention also provides a method of performing a diagnostic procedure on a patient for the purpose of determining the structure and function of tissues or organs. The method comprises administering the non-covalent carrier-hapten bioconjugates of the present invention to a patient, allowing the bioconjugates to become localized in or around a tissue or organ, and performing a diagnostic procedure such as a magnetic imaging tomographic imaging procedure.

The present invention also provides a method for altering the blood persistence of a hapten by forming the non-covalent carrier-hapten bioconjugates of the present invention. Some bioconjugates are more stable *in vivo* than the hapten alone and are cleared from the patient's blood at a slower rate than the hapten alone. The slow blood clearance rate provides more time for doing a diagnostic and therapeutic medical procedure because the hapten remains in the blood system of the patient for an extended period. Other bioconjugates are less stable *in vivo* than the hapten alone and are cleared from the patient's blood at a faster rate than the hapten alone. The faster blood clearance rate requires less time for doing a diagnostic and therapeutic medical procedure because the hapten remains in the blood system of the patient for a shorter period. This means that the patient can spend less time having the procedure and recuperating.

The following examples illustrate the specific embodiment of the invention described in this document. As will be apparent to skilled artisans, various changes and modifications are possible and are contemplated within the scope of the invention described.

### EXPERIMENTAL PROCEDURES

The following is a general procedure for the preparation and relaxivity measurements of paramagnetic bioconjugates. A mixture of the haptens **1**, **2** or **3** and the carriers polyarginine (average MW = 11,800 Daltons), 1:1 lysine-phenylalanine copolymer (average MW = 43,000), polyacrylamide-co-diallyldimethyl ammonium copolymer, serum albumin, 3:1 arginine-serine copolymer (average MW = 27,300), or styrene-maleic acid copolymer (average MW = 120,000) in water or physiological saline were kept at ambient temperature for 5 minutes and their relaxivity was measured at 40 °C using a Bruker 20 MHz spectrometer. The final concentrations of each of the species ranged from 0.1 to 1.0 mM. The relaxivities (i.e., T₁ values) of compounds **1**, **2**, **3** and **4** in pure water are 7.6, 6.2, 5.0 and 5.1, respectively. T₁ values of bioconjugates are given in the individual experimental sections.

### Example 1

### Bioconjugate of 1 and polyarginine

Concentrations of **1** and polyarginine are 0.25 mM and 0.5 mM, respectively. Relaxivity of this bioconjugate is 15.6.

### Example 2

### Bioconjugate of 1 and lysine-phenylalanine copolymer

Concentrations of **1** and lysine-phenylalanine copolymer are 0.25 mM and 0.12 mM, respectively. Relaxivity of this bioconjugate is 32.4.

### Example 3

### Bioconjugate of 1 and polyacrylamide-co-diallyldimethylammonium copolymer

Concentrations of **1** and polyacrylamide are 0.25 mM and 0.12 mM, respectively. Relaxivity of this bioconjugate is 9.6.

### Example 4

### Bioconjugate of 1 and arginine-serine copolymer

Concentrations of **1** and arginine-serine copolymer are 0.25 mM and 0.18 mM, respectively. Relaxivity of this bioconjugate is 16.0.

### Example 5

### Bioconjugate of 1 and styrene-maleic acid copolymer

Concentrations of **1** and styrene-maleic acid copolymer are 0.25 mM and 0.0003 mM, respectively. Relaxivity of this bioconjugate is 18.0.

### Example 6

### Bioconjugate of 1 and serum albumin

Concentrations of **1** and serum albumin are 0.25 mM and 0.67 mM, respectively. Relaxivity of this bioconjugate is 29.6.

### Example 7

### Bioconjugate of 2 and polyarginine

Concentrations of **2** and polyarginine are 0.5 mM and 0.5 mM, respectively. Relaxivity of this bioconjugate is 7.8.

### Example 8

### Bioconjugate of 1 and lysine-phenylalanine copolymer

Concentrations of **1** and lysine-phenylalanine copolymer are 0.5 mM and 0.12 mM, respectively. Relaxivity of this bioconjugate is 36.2.

### Example 9

### Bioconjugate of 1 and arginine-serine copolymer

Concentrations of **1** and arginine-serine copolymer are 0.5 mM and 0.18 mM, respectively. Relaxivity of this bioconjugate is 8.4.

### Example 10

### Bioconjugate of 1 and styrene-maleic acid copolymer

Concentrations of **1** and styrene-maleic acid copolymer are 0.5 mM and 0.0003 mM, respectively. Relaxivity of this bioconjugate is 11.4.

### Example 11

### Bioconiugate of 1 and serum albumin

Concentrations of **1** and serum albumin are 0.5 mM and 0.67 mM, respectively. Relaxivity of this bioconjugate is 30.0.

### Example 12

### Bioconjugate of 2 and serum albumin

Concentrations of **2** and serum albumin are 0.25 mM and 0.67 mM, respectively. Relaxivity of this bioconjugate is 29.6.

### Example 13

### Bioconjugate of 3 and styrene-maleic acid copolymer

Concentrations of **3** and styrene-maleic acid copolymer are 1.0 mM and 0.12 mM, respectively. Relaxivity of this bioconjugate is 5.5.

Although the invention has been described with respect to specific modifications, the details thereof are not to be construed as limitations, for it will be apparent that various equivalents, changes, and modifications may be resorted to without departing from the spirit and scope thereof, and it is understood that such equivalent embodiments are to be included therein.

## Claims

1. A diagnostic composition comprising a non-covalent carrier-hapten bioconjugate having the formula:
HM -----CM
wherein HM is a hapten molecule selected from the group consisting of paramagnetic metal complexes, super paramagnetic iron oxide particles, perfluorocarbons with a carbon containing portion with 1 to 20 carbon atoms, and organic free radicals with a carbon containing portion with 1 to 20 carbon atoms; CM is a neutral or charged polymeric carrier molecule selected from the group consisting of polycarboxylic acids, polyalcohols, polypeptides, polyamino acids, polyamines, polyammonium compounds, polysaccharides, polyamides, polyethers, polynucleotides, inclusion compounds, and surfactants; and the dashed line represents one or more non-covalent bonds between the carrier molecule and the hapten molecule.

2. The composition of claim 1 wherein HM is a paramagnetic metal complex.

3. The composition of claim 1 or claim 2 wherein said hapten molecule comprises a carboxylate, a sulfonate, a phosphonate, ammonium groups, phosphonium groups, lipophilic groups or a neutral electron pair donor.

4. The composition of any one of claims 1 to 3 wherein CM has a molecular weight in the range 2000 to 200000 Daltons.

5. The composition of any one of claims 1 to 4 wherein CM is selected from the group consisting of polyalcohols, polypeptides, polyaminoacids, polyamines, polyammonium compounds, and polyamides.

6. The composition of claim 5 wherein said polypeptides are selected from the group consisting of albumins, globulins, glycoproteins and lipoproteins.

7. The composition of claim 1 wherein HM is a paramagnetic metal complex comprising an element with an atomic number selected from the group consisting of atomic numbers 21-29, 42-44 and 58-70; and CM is a cationic polymer with a molecular weight range of 2000 to 200000 Daltons and is selected from the group consisting of polylysine, polyarginine, polyornithine, arginine-serine copolymer, lysine-phenylalanine copolymer, diallyldimethylammonium copolymer, and methylated bovine serum albumin.

8. The composition of claim 7 wherein CM is polylysine, polyarginine, arginine-serine copolymer, lysine-phenylalanine copolymer or polyacrylamide-co-diallyldimethyl ammonium copolymer.

9. The composition of claim 1 wherein HM is a paramagnetic metal complex comprising an element with an atomic number selected from the group consisting of atomic numbers 21-29, 42-44 and 58-70; and CM is a neutral polymer having a molecular weight range of 2000 to 200000 Daltons and is selected from the group consisting of polyalcohols, polysaccharides, polyethers, polypeptides and polyamines.

10. The composition of claim 9 wherein CM is polyhistidine, polyacrylamide-co-allyldimethylamine copolymer, polyserine or inulin.

11. The composition of claim 1 wherein HM is a paramagnetic metal complex comprising an element with an atomic number selected from the group of atomic numbers 21-29, 42-44 and 58-70; and CM is an anionic polymer having a molecular weight range of 2000 to 200000 Daltons and is selected from the group consisting of polyacrylate, polyaspartate, polyglutamate, polycarboxylates and polynucleotides.

12. The composition of claim 11 wherein CM is styrene-maleic acid copolymer, polyacrylate, polyaspartate, polyglutamate, polycarboxylate, polyadenylate, polyuridylate, polythymidylate, polyguanylate, or polycytidylate.

13. The composition of any one of claims 7 to 12 wherein HM comprises a paramagnetic complex comprising a gadolinium ion, a manganese ion or an iron ion.

14. The composition of claim 1 wherein HM comprises a paramagnetic complex comprising a gadolinium ion and CM is polyarginine.

15. A composition according to any one of claims 1 to 14, or the bioconjugate thereof, for performing a magnetic resonance diagnostic procedure.

16. A method of performing a magnetic resonance diagnostic procedure on a patient comprising administering to a patient an effective amount of a diagnostic composition according to any one of claims 1 to 14 or the bioconjugate thereof.
